# EUROPÄISCHE PATENTSCHRIFT

(11) **EP 0 569 804 B1**
(45) Veröffentlichungstag und Bekanntmachung des Hinweises auf die Patenterteilung: **08.04.1998**
(21) Anmeldenummer: 93107116.1
(22) Anmeldetag: 03.05.1993
(51) Int. Cl.: C07D 251/34, C07D 229/00, C08G 18/79

(54) **Verfahren zur Aufhellung von Isocyanurat- und Uretdiongruppen enthaltenden Polyisocyanaten**
Process for brightening polyisocyanates containing isocyanurate and uretdione groups
Procédé de blanchiment de polyisocyanates contenant des groupes isocyanurates et uretdiones

(30) Priorität: 13.05.1992 DE 4215746
(43) Veröffentlichungstag der Anmeldung: 18.11.1993
(73) Patentinhaber: BASF Aktiengesellschaft, 67063 Ludwigshafen (DE)
(72) Erfinder: Graf, Hermann, Dr., W-6704 Mutterstadt (DE); Deuker, Ernst, Dr., W-6700 Ludwigshafen (DE); Wolff, Stefan, Dr., W-6700 Ludwigshafen (DE); Schuster, Ludwig, Dr., W-6703 Limburgerhof (DE); Wack, Serge, W-6700 Ludwigshafen (DE); Hardt, Richard, W-6710 Frankenthal (DE)

(56) Entgegenhaltungen:
- EP-A- 0 377 177
- EP-A- 0 481 318

## Beschreibung

Gegenstand der Erfindung ist ein Verfahren zur Aufhellung von Isocyanurat- und Uretdiongruppen enthaltenden Polyisocyanaten.

Für hochwertige Ein- und Zweikomponenten-Polyurethanlacke mit hoher Licht- und Wetterbeständigkeit werden als Isocyanatkomponente insbesondere Isocyanurat- und Uretdiongruppen enthaltende Polyisocyanatmischungen eingesetzt.

Die Herstellung dieser Produkte erfolgt vorzugsweise durch katalytische Oligomerisierung von aliphatischen und/oder cycloaliphatischen Diisocyanaten, z.B. 1-Isocyanato-3,3,5-trimethyl-5-isocyanatomethylcyclohexan (IPDI) oder 1,6-Diisocyanatohexan (HDI).

Als Katalysatoren können beispielsweise Hydroxide oder organische Salze von schwachen Säuren mit Tetraalkylammonium-Gruppierungen, Hydroxide oder organische Salze von schwachen Säuren mit Hydroxylalkylammonium-Gruppierungen, Alkalimetallsalze oder Zinn-, Zink- bzw. Bleisalze von Alkylcarbonsäuren eingesetzt werden.

Hierbei läßt man die aliphatischen und/oder cycloaliphatischen Diisocyanate im Beisein des Katalysators, gegebenenfalls unter Verwendung von Lösungsmitteln und/oder Hilfsstoffen, bis zum Erreichen des gewünschten Umsatzes reagieren. Danach wird die Reaktion durch Desaktivierung des Katalysators abgebrochen und das überschüssige monomere Diisocyanat abdestilliert. Je nach dem verwendeten Katalysatortyp und der Reaktionstemperatur erhält man Polyisocyanate mit unterschiedlichen Anteilen an Isocyanurat- und Uretdiongruppen.

Die so hergestellten Produkte sind meist klare, aber in Abhängigkeit vom Katalysatortyp, der Diisocyanatqualität, der Reaktionstemperatur und der Reaktionsfahrweise mehr oder weniger stark gelbgefärbte Produkte.

Für die Herstellung hochwertiger Polyurethanlacke sind jedoch Produkte mit einer niedrigeren Farbzahl erwünscht. Aus dem Stand der Technik sind eine Reihe von Verfahren zur Farbzahlsenkung derartiger Produkte bekannt. So wird in DE-A-38 06 276 vorgeschlagen, den Kohlendioxidgehalt des als Monomer eingesetzten HDI vor der Oligomerisierung durch Entgasen unter Vakuum und anschließendes Durchblasen von Stickstoff durch das HDI auf weniger als 20 ppm zu senken sowie als Oligomerisierungskatalysator quaternäre Ammoniumhydroxide einzusetzen. Der Verfahrensschritt der Kohlendioxidentfernung ist jedoch technisch sehr aufwendig.

In EP-A-0 339 396 wird der Einsatz von quaternären Ammoniumfluoriden als Trimerisierungskatalysator vorgeschlagen. Bei diesem Verfahren ist zwar ein höherer Kohlendioxidgehalt tolerierbar, der eingesetzte Katalysator muß jedoch chemisch desaktiviert werden. Die daraus resultierenden Verbindungen verbleiben im Produkt und können zu anwendungstechnischen Problemen bei dessen Weiterverarbeitung führen. Eine weitere Möglichkeit zur Herstellung von Isocyanuratgruppen enthaltenden Polyisocyanaten mit niedrigerer Farbzahl besteht in dem in EP-A-0 336 205 vorgeschlagenen Zusatz von Polyesterdiolen zum Ausgangsdiisocyanat. Dadurch kann der Katalysatoreinsatz reduziert werden, die resultierenden Produkte sind jedoch immer noch vergleichsweise stark gefärbt.

Gemäß EP-A-0 377 177 werden aliphatische Diisocyanate in Gegenwart von Phosphinen als Katalysator oligomerisiert, nach Abbruch der Oligomerisierung wird das nicht umgesetzte Diisocyanat zum Teil abdestilliert, zum Teil durch Zusatz von Alkohol in Urethan umgewandelt. Anschließend wird das Reaktionsprodukt mit Peroxiden behandelt. Durch die Peroxidbehandlung kommt es zwar zu einer deutlichen Senkung der Farbzahl des Oligomerisierungsprodukts, der Einsatz von Peroxiden ist jedoch mit Problemen verbunden. So sind Peroxide oftmals technisch schwer zu handhaben. Sicherer zu handhabende Peroxide werden meist in Lösung angeboten, wobei das häufig als Lösungsmittel verwendete Dibutylphthalat bei der Lackherstellung zu anwendungstechnischen Problemen führt.

EP-A-481 318 beschreibt ein Verfahren zur Herstellung von oligomerisierten Polyisocyanaten, bei dem als Oligmerisierungskatalysatoren Verbindungen, die dreiwertigen Phosphor enthalten, eingesetzt werden.

Der Abbruch der Oligomerisierung erfolgt durch Exidation des dreiwertigen Phosphors zum fünfwertigen Phosphor durch den Einsatz von Exidationsmitteln, beispielsweise Peroxide oder auch Luft. Danach erfolgt die Abtrennung der Restmonomeren vom Reaktionsgemisch.

Die Aufgabe der Erfindung besteht darin, ein einfaches Verfahren zur wirkungsvollen Aufhellung von Isocyanurat- und Uretdiongruppen enthaltenden aliphatischen und/oder cycloaliphatischen Polyisocyanaten aufzuzeigen, das die Nachteile des Standes der Technik vermeidet.

Es wurde nun gefunden, daß diese Aufgabe überraschenderweise durch Behandlung der entmonomerisierten Isocyanurat- und Uretdiongruppen enthaltenden aliphatischen und/oder cycloaliphatischen Polyisocyanate mit Sauerstoff gelöst wird.

Gegenstand der Erfindung ist dementsprechend in Verfahren zur Aufhellung von Isocyanurat- und Uretdiongruppen enthaltenden aliphatischen und/oder cycloaliphatischen Polyisocyanaten, dadurch gekennzeichnet, daß die entmonomerisierten Isocyanurat- und Uretdiongruppen enthaltenden aliphatischen und/oder cycloaliphatischen Polyisocyanate mit Luft, reinem Sauerstoff und/oder Gemischen aus Luft oder reinem Sauerstoff mit Ozon behandelt werden.

Die Isocyanurat- und Uretdiongruppen enthaltenden Polyisocyanate werden durch an sich bekannte katalytische Oligomerisierung aliphatischer und/oder cycloaliphatischer Diisocyanate hergestellt.

Dabei können die bekannten aliphatischen und/oder cycloaliphatischen Diisocyanate eingesetzt werden, wobei für die Verarbeitung als Lackrohstoffe insbesondere IPDI und/oder HDI verwendet werden. Die Oligomerisierung wird im allgemeinen bei Temperaturen im Bereich von 0°C bis 100°C unter Durchleiten von Inertgas, vorzugsweise Stickstoff, durchgeführt. Als Katalysatoren können alle für die Oligomerisierung von aliphatischen und/oder cycloaliphatischen Diisocyanaten bekannten Katalysatoren verwendet werden, z.B. die eingangs genannten. Zur Reduzierung der Katalysatormenge ist es möglich, dem Diisocyanat vor der Oligomerisierung eine geringe Menge, bis ca. 1 Gewichtsprozent, bezogen auf das Diisocyanat, eines Diols, insbesondere eines Polyesterdiols, zuzusetzen.

Danach wird das Diisocyanat unter Rühren auf die Reaktionstemperatur gebracht und der Katalysator langsam zugegeben. Zur besseren Handhabung kann der Katalysator in einem Lösungsmittel gelöst werden. Es eignen sich hierzu beispielsweise Alkohole, insbesondere Diole, Ketone, Ether und Ester.

Nach Erreichen des gewünschten Umsetzungsgrades wird die Reaktion durch Desaktivierung des Katalysators, beispielsweise durch Zugabe eines Katalysatorgiftes oder durch thermische Zersetzung, gestoppt. Anschließend wird die Reaktionsmischung von dem überschüssigen monomeren Diisocyanat befreit. Dies geschieht bevorzugt durch Destillation, beispielsweise mittels Dünnschichtverdampfer.

Die erhaltenen Reaktionsprodukte weisen eine mehr oder weniger stark ausgeprägte Gelbfärbung auf.

Zur Reduzierung der Farbzahl werden die Isocyanurat- und Uretdiongruppen enthaltenden Polyisocyanate nun mit Sauerstoff behandelt.

Dies erfolgt am einfachsten durch Durchleiten von Sauerstoff durch das Isocyanurat- und Uretdiongruppen enthaltende Polyisocyanat.

Hierbei kann sowohl reiner Sauerstoff als auch Luft eingesetzt werden. Darüber hinaus kann der Gasstrom bis zu 20 Vol.-%, bezogen auf den Sauerstoff, an Ozon enthalten. Es hat sich außerdem als vorteilhaft erwiesen, gleichzeitig mit der Sauerstoffbehandlung eine Bestrahlung des Reaktionsprodukts mit UV-Licht durchzuführen.

Die Reaktionstemperatur sollte bei Verwendung von Luft 85°C bis 170°C, vorzugsweise 90°C bis 115°C, bei der Verwendung von Sauerstoff 80°C bis 150°C, vorzugsweise 90°C bis 110°C, bei Beimischung von Ozon 5°C bis 170°C, vorzugsweise 25°C bis 100°C betragen.

Es ist vorteilhaft, im bevorzugten mittleren Temperaturbereich zu arbeiten, da bei niedrigeren Temepraturen die Entfärbung sehr langsam vor sich geht und bei höheren Temperaturen eine teilweise Rückspaltung der Oligomeren auftreten kann. Die erfindungsgemäße Sauerstoffbehandlung wird bei Normaldruck oder leichtem Überdruck bis ca. 200 kPa durchgeführt. Die Reaktionszeiten betragen in aller Regel, abhängig von der zugeführten Sauerstoffmenge, bei der Behandlung mit Luft 0,5 bis 6 h, bei der Behandlung mit Sauerstoff 0,3 bis 3,0 h und bei Zusatz von Ozon zum Sauerstoffstrom 5 min bis 30 min. Die optimale Reaktionszeit kann durch wenige Vorversuche leicht ermittelt werden.

Daß die Aufhellung der Isocyanurat- und Uretdiongruppen enthaltenden Polyisocyanate auf diese sehr einfache Weise möglich ist, war überraschend. Der Fachmann hätte vielmehr erwarten müssen, daß eine Sauerstoffbehandlung bei den angewendeten Temperaturen zu einer Vertiefung der Farbe führen würde, zumal bekannt ist, z.B. aus JP-A-157 657, daß die Anwesenheit von Sauerstoff bei der Oligomerisierung aliphatischer Diisocyanate zu stark gefärbten Produkten führt.

Durch das erfindungsgemäße Verfahren wird es ermöglicht, Isocyanurat- und Uretdiongruppen enthaltende Polyisocyanate aufzuhellen, ohne in das Oligomerisierungsverfahren eingreifen zu müssen. Auch Oligomerisierungsprodukte mit niedrigen Farbzahlen können noch weiter aufgehellt werden.

### Beispiele

### Beispiel 1

In einem mit Rührer und Gaseinleitungsrohr versehenen Reaktionsgefäß wurden 700 g eines isocyanuratisierten HDI mit einem NCO-Gehalt von 22,3 %, einem Rest-HDI-Gehalt von 0,08 Gew.-% sowie einer Farbzahl von 63 Hazen nach DIN/ ISO 6271 vorgelegt und auf 160°C erwärmt. Bei dieser Temperatur wurde 50 Minuten lang Luft mit einer Geschwindigkeit von 5,0 l/h durch das isocyanuratisierte HDI geleitet. Danach wurde die Reaktionsmischung auf Raumtemperatur abgekühlt.

Das oligomerisierte HDI hatte nach der Behandlung einen NCO-Gehalt von 22,2 %, einen Rest-HDI-Gehalt von 0,68 Gew.-% und eine Farbzahl von 5 Hazen nach DIN/ISO 6271.

### Beispiel 2

700 g eines isocyanuratisierten HDI mit einem NCO-Gehalt von 22,3 %, einem Rest-HDI-Gehalt von 3,0 Gew.-% und einer Farbzahl von 100 Hazen nach DIN/ISO 6271 wurden in ein Reaktionsgefäß analog Beispiel 1 eingefüllt und auf eine Temperatur von 158°C bis 162°C erwärmt. Danach wurde 40 Minuten lang Luft mit einer Geschwindigkeit von 5,0 l/h durch das isocyanuratisierte HDI geleitet. Nach der Abkühlung betrug die Farbzahl 35 Hazen nach DIN/ISO 6271.

### Beispiel 3

700 g isocyanuratisiertes HDI mit einem NCO-Gehalt von 22,3 %, einem Rest-HDI-Gehalt von 2,8 % und einer Farbzahl von 35 Hazen nach DIN/ISO 6271 wurden in ein Reaktionsgefäß analog Beispiel 1 gegeben und auf 131°C aufgeheizt. Bei dieser Temperatur wurde 40 Minuten lang Luft mit einer Geschwindigkeit von 30 l/h durch das isocyanuratisierte HDI geleitet. Nach der Abkühlung betrug seine Farbzahl 15 Hazen nach DIN/ISO 6271.

### Beispiel 4

50 g eines isocyanuratisierten HDI wurden in ein Reaktionsgefäß analog Beispiel 1 gegeben und auf 120°C erwärmt. Bei dieser Temperatur wurde 25 Minuten lang Luft mit einer Geschwindigkeit von 3,0 l/h durch das isocyanuratisierte HDI geleitet. Dabei sank die Farbzahl von 14 Hazen auf 2 Hazen nach DIN/ISO 6271.

### Beispiel 5

Es wurde wie in Beispiel 4 verfahren, jedoch die Reaktionstemperatur auf 100°C gehalten. Die Farbzahl des isocyanuratisierten HDI sank von 16 Hazen auf 11 Hazen nach DIN/ ISO 6271.

### Beispiel 6

Es wurde wie in Beispiel 4 verfahren, jedoch mit einer Reaktionstemperatur von 90°C und einer Reaktionsdauer von 95 Minuten. Die Farbzahl des isocyanuratisierten HDI sank von 31 Hazen auf 17 Hazen nach DIN/ISO 6271.

### Beispiel 7

In einem kontinuierlich betriebenen Rührkessel, der mit 400 kg/h eines isocyanuratisierten HDI mit einem Rest-HDI-Gehalt von 4 % und einer Farbzahl von 80 Hazen nach DIN/ ISO 6271 beschickt wurde, leitete man 20 m³/h Luft so ein, daß die Flüssigkeit intensiv durchspült wurde. Die mittlere Verweilzeit des isocyanuratisierten HDI betrug 2,8 Stunden, die Reaktionstemperatur 120°C. Die Farbzahl des austretenden Produkts betrug 30 Hazen nach DIN/ISO 6271.

### Beispiel 8

Es wurde wie in Beispiel 7 verfahren, jedoch betrug die mittlere Verweilzeit 2,5 Stunden und die Reaktionstemperatur 110°C. Die Farbzahl des isocyanuratisierten HDI sank von 60 Hazen auf 25 Hazen nach DIN/ISO 6271.

### Beispiel 9

Es wurde wie in Beispiel 7 verfahren, jedoch betrug der Rest-HDI-Gehalt des isocuanuratisierten HDI 2,0 Gew.-%. Die Farbzahl sank von 80 Hazen auf 40 Hazen nach DIN/ISO 6271.

### Beispiel 10

In einem kontinuierlich betriebenen Rührkessel, der mit 360 kg/h eines isocyanuratisierten HDI mit einem NCO-Gehalt von 22,2 %, einem Rest-HDI-Gehalt von 0,08 Gew.-% und einer Farbzahl von 60 Hazen nach DIN/ISO 6271 beschickt wurde, leitete man 20 m³/h Luft so ein, daß die Flüssigkeit intensiv durchströmt wurde. Die mittlere Verweilzeit des isocyanuratisierten HDI betrug 5,8 Stunden, die Reaktionstemperatur 95°C. Das aus dem Rührkessel austretende Produkt hatte eine Farbzahl von 15 Hazen nach DIN/ISO 6271.

### Beispiel 11

Es wurde verfahren wie in Beispiel 10, jedoch mit 370 kg/h Produkteinspeisung, 5,6 h mittlerer Verweilzeit und 90°C Reaktionstemperatur. Die Farbzahl des isocanuratisierten HDI sank von 60 Hazen auf 25 Hazen nach DIN/ISO 6271.

### Beispiel 12

In einem 500-ml-Reaktionsgefäß, ausgerüstet mit einem Begasungsrührer mit einer maximalen Drehzahl von 2000 Umdrehungen pro Minute, wurden 330 g eines isocyanuratisierten HDI mit einem NCO-Gehalt von 22,4 %, einem Rest-HDI-Gehalt von 0,08 Gew.-% und einer Farbzahl von 35 Hazen nach DIN/ ISO 6271 vorgelegt und auf 90°C aufgeheizt. Bei dieser Temperatur wurde über den Rührer 7 Minuten lang ein Ozon/ Sauerstoff-Gemisch mit einem Ozongehalt von 250 g Ozon pro m³ Sauerstoff mit einer Geschwindigkeit von 50 l/h eingeleitet. Die Farbzahl des isocyanuratisierten HDI betrug nach der Behandlung weniger als 5 Hazen nach DIN/ISO 6271, die übrigen Produktkennzahlen blieben unverändert.

### Beispiel 13

Es wurde verfahren wie in Beispiel 12, jedoch hatte das isocyanuratisierte HDI einen NCO-Gehalt von 22,3 %, einen Rest-HDI-Gehalt von 3,0 Gew.-% und eine Farbzahl von 40 Hazen nach DIN/ISO 6271, und die Reaktionszeit betrug 10 Minuten. Die Farbzahl des isocyanuratisierten HDI betrug nach der Behandlung 5 Hazen nach DIN/ISO 6271, die übrigen Produktkennzahlen blieben unverändert.

### Beispiel 14

In einem 3,4-l-Reaktor, der mit einem in Beispiel 12 beschriebenen Begasungsrührer ausgerüstet war, wurden 3,1 kg isocyanuratisiertes HDI, dem 0,03 Gew.-% Bezoylchlorid zugesetzt worden war, auf 70°C erwärmt. Bei dieser Temperatur wurde über den Begasungsrührer 15 Minuten lang ein Ozon/ Sauerstoff-Gemisch mit einem Ozongehalt von 240 g Ozon pro m³ Sauerstoff und einer Geschwindigkeit von 50 l/h eingeleitet. Dabei sank die Farbzahl des isocyanuratisierten HDI von 40 Hazen auf 6 Hazen nach DIN/ISO 6271.

### Beispiel 15

Es wurde wie in Beispiel 12 verfahren, jedoch mit einer Reaktionstemperatur von 90°C, einer Ozonkonzentration von 44 g Ozon pro m³ Sauerstoff und einer Reaktionszeit von 10 Minuten. Die Farbzahl des isocyanuratisierten HDI sank von 50 Hazen auf 6 Hazen nach DIN/ISO 6271.

### Beispiel 16

800 g eines isocyanuratisierten HDI mit einem NCO-Gehalt von 22,4 %, einem Restmonomerengehalt von 0,10 Gew.-% und einer Farbzahl von 128 Hazen nach DIN/ISO 6271 wurden in ein mit einem Gaseinleitungsrohr und einem Rührer vesehenes Reaktionsgefäß eingefüllt und auf 90°C erwärmt. Bei dieser Temperatur wurde 13 l/h Sauerstoff mit einer Reinheit von 99,9 % durch das Gaseinleitungsrohr in die Flüssigkeit eingeperlt. Die Farbzahl des isocyanuratisierten HDI betrug nach 30 Minuten Reaktionszeit 86 Hazen, nach 60 Minuten Reaktionszeit 62 Hazen und nach 210 Minuten Reaktionszeit 37 Hazen. Die übrigen Produktkennzahlen blieben unverändert.

### Beispiel 17

Es wurde verfahren wie in Beispiel 16, jedoch hatte das isocyanuratisierte HDI eine Farbzahl von 94 Hazen nach DIN/ ISO 6271, und die Reaktionstemperatur betrug 105°C. Die Farbzahl des isocyanuratisierten HDI betrug nach 30 Minuten 41 Hazen, nach 60 Minuten 29 Hazen, nach 90 Minuten 21 Hazen und nach 120 Minuten 8 Hazen nach DIN/ISO 6271.

### Beispiel 18

30 g eines isocyanuratisierten HDI mit einem NCO-Gehalt von 23,3 %, einem Restmonomerengehalt von 3,2 Gew.-% und einer Farbzahl von 51 Hazen wurden unter Luftzutritt auf 161°C erwärmt. Tabelle 1 gibt die Abhängigkeit der Farbzahl von der Reaktionszeit wieder.

| | | | | | | | |
|---|---|---|---|---|---|---|---|
| Zeit (min) | 0 | 10 | 15 | 20 | 30 | 50 | 70 |
| Farbzahl (Hazen) | 51 | 20 | 11 | 7 | 14 | 34 | 51 |

Das isocyanuratisierte HDI hatte nach Versuchsende folgende Produktkennzahlen: NCO-Gehalt 22,4 %, Restmonomerengehalt 3,1 Gew.-%.

## Patentansprüche

1. Verfahren zur Aufhellung von entmonomerisierten Isocyanurat- und Uretdiongruppen enthaltenden aliphatischen und/oder cycloaliphatischen Polyisocyanaten in Anwesenheit eines Oxidationsmittels, dadurch gekennzeichnet, daß als Oxidationsmittel Luft, reiner Sauerstoff oder Gemische aus Luft oder reinem Sauerstoff mit Ozon durch die entmonomerisierten Isocyanurat- und Uretdiongruppen enthaltenden Polyisocyanate geleitet werden.

2. Verfahren nach Anspruch 1, dadurch gekennzeichnet, daß als Isocyanurat- und Uretdiongruppen enthaltende Polyisocyanate isocyanuratisierte aliphatische und/oder cycloaliphatische Diisocyanate eingesetzt werden.

3. Verfahren nach Anspruch 1 oder 2, dadurch gekennzeichnet, daß als isocyanuratisiertes aliphatisches Diisocyanat isocyanuratisiertes 1,6-Hexamethylendiisocyanat eingesetzt wird.

4. Verfahren nach einem der Ansprüche 1 bis 3, dadurch gekennzeichnet, daß die Sauerstoffbehandlung mit Luft durchgeführt wird.

5. Verfahren nach einem der Ansprüche 1 bis 3, dadurch gekennzeichnet, daß die Sauerstoffbehandlung mit reinem Sauerstoff durchgeführt wird.

6. Verfahren nach einem der Ansprüche 1 bis 5, dadurch gekennzeichnet, daß der Sauerstoff- bzw. Luftstrom bis zu 20 Volumen-% Ozon, bezogen auf den Sauerstoff, enthält.

7. Verfahren nach einem der Ansprüche 1 bis 4, dadurch gekennzeichnet, daß die Behandlung mit Luft bei Temperaturen von 85°C bis 170°C durchgeführt wird.

8. Verfahren nach Anspruch 7, dadurch gekennzeichnet, daß die Behandlung mit Luft bei Temperaturen von 90°C bis 115°C erfolgt.

9. Verfahren nach einem der Ansprüche 1 bis 3 und 5, dadurch gekennzeichnet, daß die Behandlung mit reinem Sauerstoff bei Temperaturen von 80°C bis 150°C durchgeführt wird.

10. Verfahren nach Anspruch 9, dadurch gekennzeichnet, daß die Behandlung mit reinem Sauerstoff bei Temperaturen von 90°C bis 110°C durchgeführt wird.

11. Verfahren nach einem der Ansprüche 1 bis 3 und 6, dadurch gekennzeichnet, daß die Behandlung bei Zusatz von Ozon zum Sauerstoff- bzw. Luftstrom bei Temperaturen von 5°C bis 170°C durchgeführt wird.

12. Verfahren nach Anspruch 11, dadurch gekennzeichnet, daß die Behandlung bei Zusatz von Ozon zum Sauerstoff- bzw. Luftstrom bei Temperaturen von 15°C bis 100°C durchgeführt wird.

## Claims

1. A process for decoloring demonomerized aliphatic and/or cycloaliphatic polyisocyanates containing isocyanurate and uretdione groups in the presence of an oxidant, wherein the oxidant in the form of air, pure oxygen or a mixture of air or pure oxygen with ozone, is passed through the demonomerized polyisocyanates containing isocyanurate and uretdione groups.

2. A process as claimed in claim 1, wherein the polyisocyanates containing isocyanurate and uretdione groups are aliphatic and/or cycloaliphatic diisocyanates which have been modified with isocyanurate groups.

3. A process as claimed in claim 1 or 2, wherein the aliphatic diisocyanate which has been modified with isocyanurate groups is 1,6-hexamethylene diisocyanate which has been modified with isocyanurate groups.

4. A process as claimed in claim 1 or 2 or 3, wherein the oxygen treatment is carried out by means of air.

5. A process as claimed in claim 1 or 2 or 3, wherein the oxygen treatment is carried out by means of pure oxygen.

6. A process as claimed in claim 1 or 2 or 3 or 4 or 5, wherein the stream of oxygen or air contains up to 20% by volume of ozone, based on the oxygen.

7. A process as claimed in claim 1 or 2 or 3 or 4, wherein the treatment is carried out with air at from 85 to 170°C.

8. A process as claimed in claim 7, wherein the treatment is carried out with air at from 90 to 115°C.

9. A process as claimed in claim 1 or 2 or 3 or 5, wherein the treatment is carried out with pure oxygen at from 80 to 150°C.

10. A process as claimed in claim 9, wherein the treatment is carried out with pure oxygen at from 90 to 110°C.

11. A process as claimed in claim 1 or 2 or 3 or 6, wherein the treatment is carried out at from 5 to 170°C with addition of ozone to the stream of oxygen or air.

12. A process as claimed in claim 11, wherein the treatment is carried out at from 15 to 100°C with the addition of ozone to the stream of oxygen or air.

## Revendications

1. Procédé pour le blanchiment de polyisocyanates aliphatiques et/ou cycloaliphatiques contenant des groupes isocyanurates et uretdiones, dont on a enlevé les monomères, en présence d'un agent d'oxydation, caractérisé en ce qu'on guide, à titre d'agents d'oxydation, de l'air, de l'oxygène pur ou des mélanges d'air et d'oxygène pur avec de l'ozone à travers les polyisocyanates contenant des groupes isocyanurates et uretdiones, dont on a enlevé les monomères.

2. Procédé selon la revendication 1, caractérisé en ce qu'on met en oeuvre, à titre de polyisocyanates contenant des groupes isocyanurates et uretdiones, des diisocyanates aliphatiques et/ou cycloaliphatiques isocyanuratisés.

3. Procédé selon la revendication 1 ou 2, caractérisé en ce qu'on met en oeuvre, à titre de diisocyanate aliphatique isocyanuratisé, le 1,6-hexaméthylènediisocyanate isocyanuratisé.

4. Procédé selon l'une quelconque des revendications 1 à 3, caractérisé en ce qu'on effectue le traitement à l'oxygène avec de l'air.

5. Procédé selon l'une quelconque des revendications 1 à 3, caractérisé en ce qu'on effectue le traitement à l'oxygène avec de l'oxygène pur.

6. Procédé selon l'une quelconque des revendications 1 à 5, caractérisé en ce que le courant d'oxygène, respectivement le courant d'air contient de l'ozone jusqu'à concurrence de 20% en volume rapportés à l'oxygène.

7. Procédé selon l'une quelconque des revendications 1 à 4, caractérisé en ce qu'on effectue le traitement avec de l'air à des températures de 85°C à 170°C.

8. Procédé selon la revendication 7, caractérisé en ce qu'on effectue le traitement avec de l'air à des températures de 90°C à 115°C.

9. Procédé selon l'une quelconque des revendications 1 à 3 et 5, caractérisé en ce qu'on effectue le traitement avec de l'oxygène pur à des températures de 80°C à 150°C.

10. Procédé selon la revendication 9, caractérisé en ce qu'on effectue le traitement avec de l'oxygène pur à des températures de 90°C à 110°C.

11. Procédé selon l'une quelconque des revendications 1 à 3 et 6, caractérisé en ce qu'on effectue le traitement par addition d'ozone à un courant d'oxygène, respectivement d'air, à des températures de 5°C à 170°C.

12. Procédé selon la revendication 11, caractérisé en ce qu'on effectue le traitement par addition d'ozone à un courant d'oxygène, respectivement d'air, à des températures de 15°C à 100°C.
